(19) Europäisches Patentamt
European Patent Office
Office européen des brevets

(11) **EP 1 923 381 B1**

(12) **EUROPEAN PATENT SPECIFICATION**

(45) Date of publication and mention
of the grant of the patent:
**09.03.2011 Bulletin 2011/10**

(51) Int Cl.:
**C07C 51/487** *(2006.01)*    **C07C 63/38** *(2006.01)*

(21) Application number: **07013628.8**

(22) Date of filing: **11.07.2007**

(54) **Hydrogenation process for high-purity naphthalenedicarboxylic acid**

Hydrierungsverfahren für hochreine Naphthalindicarbonsäure

Procédé d'hydrogénation pour acide naphtalènedicarboxylique de pureté élevée

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **16.11.2006 KR 20060113255**

(43) Date of publication of application:
**21.05.2008 Bulletin 2008/21**

(73) Proprietor: **Hyosung Corporation**
**Dongan-ku**
**Anyang-si**
**Kyonggi do 431-080 (KR)**

(72) Inventors:
• **Kim, Hyun-Soo**
**Anyang-si**
**Kyonggi-do 431-080 (KR)**
• **Choi, Young-Gyo**
**Anyang-si**
**Kyonggi-do 431-080 (KR)**

(74) Representative: **Beier, Ralph**
**v. Bezold & Partner**
**Akademiestrasse 7**
**80799 München (DE)**

(56) References cited:
**EP-A- 1 306 361    WO-A-99/29652**

Remarks:
The file contains technical information submitted after the application was filed and not included in this specification

## Description

Technical Field

The present invention relates to a process for preparing high purity 2,6-naphthalenedicarboxylic acid (hereinafter referred to as "PNDA") by a selective hydrogenation reaction in order to remove impurities included in crude 2,6-naphthalenedicarboxylic acid (hereinafter referred to as "CNDA").

2,6-naphthalenedicarboxylic acid is used as a monomer for preparing polyethylenenaphthalate(PEN) from which high functional fibers and film are prepared. In particular, since mechanical, thermal and chemical properties of PEN are superior to those of polyethyleneterephthalate (PET), the 2,6-naphthalenedicarboxylic acid is used in a wide variety of commercial applications, for example, films, fibers, insulators, magnetic tapes and beverage packaging applications.

The above-mentioned CNDA is prepared by an oxidization reaction of 2,6-dimethylnaphthalene (DMN) in the presence of a heavy metal catalyst. The CNDA produced from the oxidization reaction, however, contains a large amount of various impurities as by-products such as naphthoic acid (NA), formyl-naphthoic acid (FNA), methylnaphthoic acid (MNA), trimellitic acid (TMLA), naphthalenecarboxylate bromide (Br-NDA) and high molecule organic impurities(heavy substances). If the CNDA having such impurities is polymerized with ethylene glycol, the quality of the PEN is seriously degraded by lowering thermal resistance and softening point and causing colorization. Accordingly, a high purity PNDA is required to obtain high quality PEN.

Background Art

Under these circumstances, many of hydrogenation reaction processes to obtain high purity PNDA have been suggested in prior arts. For example, US Patent No. 5,256,817 discloses a process for preparing high purity NDA using an acetic acid or an aqueous solution of acetic acid as a solvent at 315~371°C of reaction temperature. US Patent No. 6,756,509 suggests using water as solvent, in which hydrogen of 10~100 ppm and the CNDA are melted in the water, and then the resultant solution is input into a fixed-bed catalytic reactor at 280~350°C and a saturated vapor pressure of 152~253 x $10^5$ Pa (150~250 atm). In the process, the FNA is removed from the reaction resultant, which is then washed with ethanol to obtain a high purity NDA. Furthermore, US Patent 6,747,171 discloses another process using water or aqueous solution of acetic acid as a solvent at 271~301°C in the presence of catalyst of palladium (Pd) of a Group 8 element and stannum (Sn) of a Group 4B supported on activated carbon.

In the above-mentioned hydrogenation reaction processes, however, the amount of hydrogen was not quantitatively input relative to impurities in the reactor, and consequently, impurities were not completely removed. Furthermore, dicarboxylic tetraline (DCT) is produced as a by-product, which results in degrading a purity improvement effect. As the number of washing increases, the yield is lowered, and the reaction becomes inefficient due to the use of expensive washing solvents instead of water.

EP 1 306 361 A1 describes a process for producing purified naphthalenedicarboxylic acid from a raw mixture of crude terephthalic acid and crude naphthalenedicarboxylic acid wherein the content of crude naphthalenedicarboxylic acid is 0.1 to 10 mass percent of the crude terephthalic acid content. Said process involves a hydrogenation step wherein the amount of hydrogen added is preferably in the range of from 1 to 10 mole percent of the total of the terephthalic acid and the naphthalenedicarboxylic acid in the raw mixture.

WO 99/29652 discloses a hydrogenation process for removing impurities from crude 2,6-naphthalenedicarboxylic acid wherein the amount of hydrogen used in said hydrogenation is based on the mole ratio of aldehyde, such as 2-formyl-6-naphthoic acid (2-20 times the amount of FNA). In this process, further contaminants, namely dicarboxytetralins (DCTs) are produced.

It is an object of the present invention to provide an improved process of hydrogenation reaction to solve the above-mentioned problems. Accordingly, the present invention provides a method of inputting a fixed-quantity of hydrogen in the hydrogenation reactions of FNA, Br-NDA and high molecule heavy substances contained in the CNDA produced from the oxidization reaction wherein the fixed-quantity of hydrogen is calculated according to formula 1 of claim 1.

Brief Description of the Drawings

The above and other objects, features and advantages of the present invention will be apparent from the following detailed description of the preferred embodiments of the invention in conjunction with the accompanying drawings, wherein:

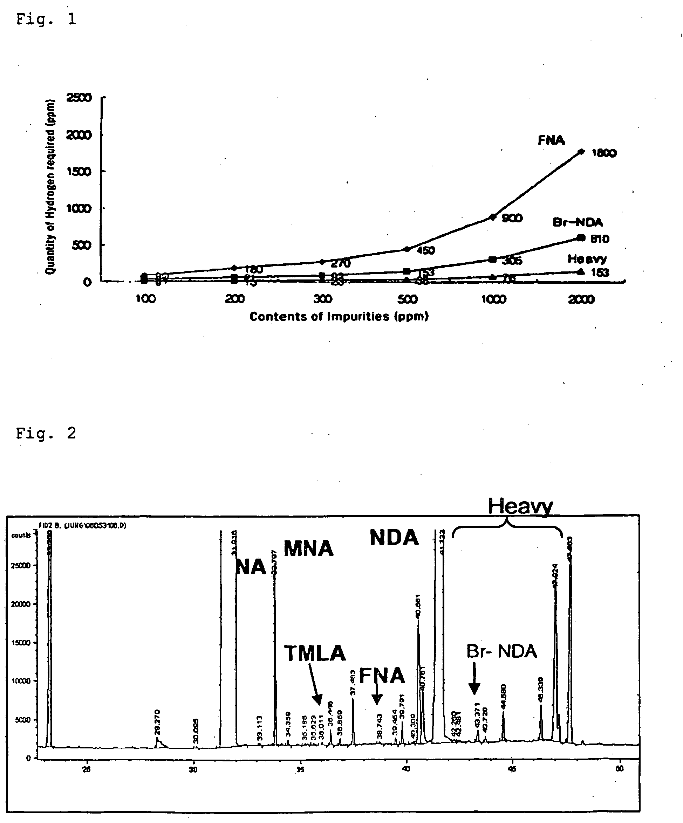

Fig.1 is an illustration of required amounts of hydrogen depending on impurities according to the present invention;
Fig.2 is a gas chromatography analysis of the compositions of impurities measured before the hydrogenation reaction according to the present invention;

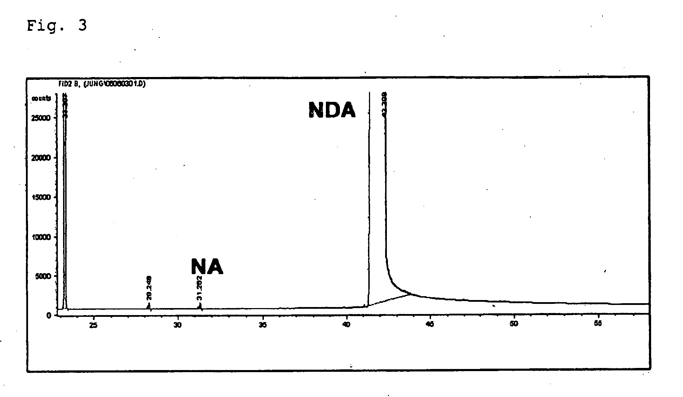

Fig.3 is a gas chromatography analysis of the compositions of impurities measured after the hydrogenation reaction according to the present invention.

Disclosure of the Invention

According to the present invention, a formula of calculating a fixed-quantity of hydrogen is provided in order to prepare high purity 2,6-naphthalenedicarboxylic acid, wherein the fixed-quantity of hydrogen is acquired for the hydrogenation reactions of FNA, Br-NDA and high molecule heavy substances contained in the CNDA obtained from the oxidization reaction.

According to the present invention, a fixed-quantity of hydrogen precalculated according to the following formula 1 is added into a reactor in order to remove FNA, Br-NDA and heavy substances.

[Formula 1]

$$\text{Quantity of hydrogen required for removing FNA (ppm/hr)}$$

$$= \frac{[\text{CNDA input (g/hr)}] \times [\text{FNA content(ppm)}] \times [2H_2 (1/mol)]}{[\text{FNA MW (g/mol)}]}$$

$$\text{Quantity of hydrogen required for removing Br-NDA (ppm/hr)}$$

$$= \frac{[\text{CNDA input (g/hr)}] \times [\text{Br-NDA content(ppm)}] \times [H_2 (1/mol)]}{[\text{Br-NDA MW (g/mol)}]}$$

$$\text{Quantity of hydrogen required for removing heavy substances (ppm/hr)}$$

$$= \frac{[\text{CNDA input (g/hr)}] \times [\text{heavy substance content(ppm)}] \times [1/2H_2 (1/mol)]}{[\text{NDA MW (g/mol)}]}$$

According to another preferred embodiment of the present invention, the hydrogenation reaction is carried out at 90Kg/cm$^2$~130Kg/cm$^2$ and 290~315°C in a liquid phase.

According to another preferred embodiment of the present invention, the hydrogen reaction is carried out in the presence of a catalyst containing palladium or platinum of 0.4~0.6 wt% based on element weights supported on activated carbon.

According to another preferred embodiment of the present invention, the product obtained from the hydrogenation reaction of 2,6-naphthalenedicarboxylic acid is washed with 200~300°C of hot water one time in order to prepare high purity 2,6-naphthalenedicarboxylic acid.

Hereinafter, the present invention will be described in detail.

The impurities such as FNA, Br-NDA and heavy substances contained in the CNDA produced by the oxidization reaction of 2,6-dimethylnnaphthalene(DMN) is removed by a reduction process using hydrogen. It has not clearly known about an exact molecule structure of heavy substances, but it is presumed to be isomer or complex of NDA. Accordingly, inventors of the present invention had paid attention to these presumptions. Inventors calculated the amount of hydrogen based on the molecular weight of NDA required for removing the heavy substances, and used 1/2 mol of hydrogen per 1 mol of NDA in order to lower a possibility of NDA decomposition caused by excessive amount of hydrogen. As a result, they found this process efficiently removed heavy substances. As shown in the following reaction formula, each amount of hydrogen required for hydrogenation reactions of FNA, Br-NDA and heavy substances in the reactor is 2 mol, 1 mol and 1/2 mol per mol of each component.

[Reaction formula of impurities and hydrogen]

FNA + 2H$_2$ --> MNA + H$_2$O
BR-NDA + H$_2$ --> NDA + HBr
Heavy substances + 1/2H$_2$ --> NA + light

Each fixed-quantity of hydrogen required for removing each impurity is calculated according to the following formula 1, and then quantitatively input into a reactor using hydrogen MFC (Mass Flow Controller) in the presence of a hydrogenation catalyst.

[Formula 1]

$$\text{Quantity of hydrogen required for removing FNA (ppm/hr)} = \frac{[\text{CNDA input (g/hr)}] \times [\text{FNA content(ppm)}] \times [2H_2 (1/\text{mol})]}{[\text{FNA MW (g/mol)}]}$$

$$\text{Quantity of hydrogen required for removing Br-NDA (ppm/hr)} = \frac{[\text{CNDA input (g/hr)}] \times [\text{Br-NDA content(ppm)}] \times [H_2 (1/\text{mol})]}{[\text{Br-NDA MW (g/mol)}]}$$

$$\text{Quantity of hydrogen required for removing heavy substances (ppm/hr)} = \frac{[\text{CNDA input (g/hr)}] \times [\text{heavy substance content(ppm)}] \times [1/2 H_2 (1/\text{mol})]}{[\text{NDA MW (g/mol)}]}$$

The catalyst containing palladium or platinum of 0.4~0.6 wt% based on element weight as activated components and supported on activated carbon may be preferably used for hydrogenation reaction.

The hydrogenation reaction may be preferably carried out at 90~130 Kg/cm$^2$ and 290~315°C in a liquid phase. It is more preferable to carry out under the condition of 310~312°C and 100~110 Kg/cm$^2$. The homogeneous liquid-phase reaction may be maximized in reaction effect. Considering that solubility is determined according to the concentration of hydrogen and NDA (e.g. the solubility of NDA relative to 100g of water is 10g at 308°C), it is required to set the temperature and pressure to carry out the hydrogenation reaction in a liquid phase. Under these conditions, impurities in the CNDA are successfully removed. Accordingly, it is the most preferable to set the temperature and pressure so as to dissolute 7~10g of NDA. The optimal condition is 308 °C and 105 Kg/cm$^2$.

The product obtained from the reduction reaction of CNDA is washed with water. It is preferable to use 200~300 °C of hot water as a washing solvent. It is more preferable to use 225~240 °C of water in order to minimize the loss of NDA. The number of washing may be one or more. But according to the present invention wherein the hydrogen is quantitatively input in the hydrogenation reaction, a high purity NDA may be prepared with just one time of washing.

Hereinafter, the present invention will be described in detail with Examples. These examples are provided only for the illustrative purpose, and it should not be construed that the scope of the invention is limited thereto.

EXAMPLES

[Comparative Example 1 to 3 and Example 1]

The CDNA obtained from the oxidization reaction of 2,6-dimethylnaphthalene is input into a hydrogenation reactor at a pressure of 4.8 Kg/hr. And hydrogen is input into the hydrogenation reactor by 50, 80 and 100 ppm/hr. According to the formula in the present invention, 156.7 ppm/hr of hydrogen is calculated and then input into the reactor. The reaction temperature and pressure are 308°C and 105 Kg/cm$^2$, respectively.
The result is shown in Table 1.

[Table 1]

| Composition before reaction | | Composition after reaction | | | |
|---|---|---|---|---|---|
| | | Hydrogen input(ppm/hr) | | | |
| | | Comp. Ex. 1 | Comp. Ex. 2 | Comp. Ex. 3 | Ex. 1 |
| | | 50 | 80 | 100 | 156.7 |
| NDA(wt%) | 99.599 | 99.634 | 99.697 | 99.741 | 99.874 |
| FNA(ppm) | 1071 | 751 | 552 | 454 | 0 |
| Br-NDA(ppm) | 1332 | 820 | 341 | 297 | 0 |
| Heavy(ppm) | 647 | 649 | 478 | 390 | 0 |

[Example 2 to 11]

The CDNA is input into the hydrogenation reactor at a pressure of 4.8 Kg/hr, and a fixed-quantity of hydrogen relative to the amount of impurities contained the CNDA is calculated according to the formula of the present invention and then input into the hydrogen reactor. The reaction temperature and pressure are 298°C and 100 Kg/cm$^2$, respectively.
The result is shown in Table 2.

[Table 2]

| Example | Concentration of impurities before reaction (ppm) | | Hydrogen input (ppm/hr) | Concentration of impurities after reaction (ppm) | |
|---|---|---|---|---|---|
| 2 | FNA | 641 | 107.3 | FNA | N.D |
| | Br-NDA | 727 | | Br-NDA | N.D |
| | Heavy | 1357 | | Heavy | N.D |
| 3 | FNA | 610 | 79.1 | FNA | N.D |
| | Br-NDA | 310 | | Br-NDA | N.D |
| | Heavy | 642 | | Heavy | N.D |
| 4 | FNA | 777 | 99.3 | FNA | N.D |
| | Br-NDA | 259 | | Br-NDA | N.D |
| | Heavy | 1004 | | Heavy | N.D |
| 5 | FNA | 630 | 161.7 | FNA | N.D |
| | Br-NDA | 2060 | | Br-NDA | N.D |
| | Heavy | 2101 | | Heavy | N.D |
| 6 | FNA | 1391 | 200.2 | FNA | N.D |
| | Br-NDA | 1288 | | Br-NDA | N.D |
| | Heavy | 1521 | | Heavy | N.D |

(continued)

| Example | Concentration of impurities before reaction (ppm) | | Hydrogen input (ppm/hr) | Concentration of impurities after reaction (ppm) | |
|---|---|---|---|---|---|
| 7 | FNA | 2986 | 328.6 | FNA | N.D |
| | Br-NDA | 1027 | | Br-NDA | N.D |
| | Heavy | 523 | | Heavy | N.D |
| 8 | FNA | 1497 | 223.3 | FNA | N.D |
| | Br-NDA | 2183 | | Br-NDA | N.D |
| | Heavy | 4765 | | Heavy | N.D |
| 9 | FNA | 5560 | 576.3 | FNA | N.D |
| | Br-NDA | 767 | | Br-NDA | N.D |
| | Heavy | 1103 | | Heavy | N.D |
| 10 | FNA | 690 | 105.1 | FNA | N.D |
| | Br-NDA | 358 | | Br-NDA | N.D |
| | Heavy | 1671 | | Heavy | N.D |
| 11 | FNA | 546 | 86.0 | FNA | N.D |
| | Br-NDA | 300 | | Br-NDA | N.D |
| | Heavy | 1462 | | Heavy | N.D |

Washing process

The resultant solution obtained from the hydrogenation reactor is crystallized in a 70L of crystallyzer, and then moved to a washing device to be washed with 40kg of hot water (200~300°C), filtered and dried. Each hydrogenated product in Comparative Examples 1 to 3 and Examples 1 and 11 is washed with 225°C of hot water, and the result is shown in Table 3. The purity of the product is measured using gas chromatography to compare purities before and after washing. The result is shown in the Table 3. According to the Table 3, the purities measured before washing in Example 1 and 11 are higher than those in Comparative Examples. In other words, the fixed-quantity input of hydrogen to remove impurities resulted in higher purity measured before washing and, consequently, lower number of washing.

[Table 3]

| | Comp. Ex. 1 | Comp. Ex. 2 | Comp. Ex. 3 | Ex. 1 | Ex. 11 |
|---|---|---|---|---|---|
| Purity (%) (before washing) | 99.63 | 99.70 | 99.74 | 99.87 | 99.82 |
| The number of washing | 7 | 5 | 5 | 1 | 1 |
| Purity (%) (after washing | 99.87 | 99.88 | 99.88 | 99.94 | 99.91 |

Effects of Invention

According to the present invention, the impurities of NDA such as FNA, Br-NDA and heavy substances were almost all removed as a result of the quantitative inputting of hydrogen calculated by the formulas in Example 1 to 11. A gas chromatography analysis of composition of impurities before and after the hydrogenation reaction shows the same result. Accordingly, it is an advantage of the present invention to obtain higher purity NDA before washing process compared to conventional arts, and consequently, to decrease the number and the cost of washing. As shown in the Table 3, it was required to conduct five and more times of washing in Comparative Example 1 to 3, while it is enough to carry out just one time of washing in Example 1 to 11 to obtain higher purity NDA. Furthermore, the decreased number of washing process may result in minimizing the NDA loss and increasing the yield.

**Claims**

1. A process of removing by hydrogenation impurities including formylnaphthoic acid, naphthalenecarboxylate bromide and high molecule organic impurities from a solution of crude 2,6-naphthalenedicarboxylic acid (CNDA), the process comprising the steps of;

   (a) calculating an amount of hydrogen required to remove impurities such as formylnaphthoic acid, naphthalenecarboxylate bromide and high molecule organic impurities of heavy substances;
   (b) inputting a fixed quantity of hydrogen as calculated in step (a) and removing the impurities by hydrogenation; and
   (c) washing the product obtained from the hydrogenation reaction of step (b) with 200 to 300°C hot water one time to produce purified 2,6-naphthalenedicarboxylic acid,

   wherein the fixed quantity of hydrogen is calculated according to a following formula 1

   [Formula 1]

   Quantity of hydrogen required for removing FNA (ppm/hr)

   $$= \frac{[\text{CNDA input (g/hr)}] \times [\text{FNA content(ppm)}] \times [2\text{H}_2(1/\text{mol})]}{[\text{FNA MW (g/mol)}]}$$

   Quantity of hydrogen required for removing Br-NDA (ppm/hr)

   $$= \frac{[\text{CNDA input (g/hr)}] \times [\text{Br-NDA content(ppm)}] \times [\text{H}_2(1/\text{mol})]}{[\text{Br-NDA MW (g/mol)}]}$$

   Quantity of hydrogen required for removing heavy substances (ppm/hr)

   $$= \frac{[\text{CNDA input (g/hr)}] \times [\text{heavy substance content(ppm)}] \times [1/2\text{H}_2(1/\text{mol})]}{[\text{NDA MW (g/mol)}]}$$

2. The process according to claim 1, wherein the hydrogenation reaction is carried out under 90 to 130 kg/cm$^2$ and 290 to 315°C in a liquid phase.

3. The process according to claim 1, wherein the hydrogen reaction is carried out in the presence of a catalyst supported on activated carbon and containing 0.4 to 0.6 wt% palladium or platinum based on an element weight as activated components.

**Patentansprüche**

1. Verfahren zur Entfernung von Verunreinigungen, einschließlich Formylnaphthalincarbonsäure (FNA), Naphthalincarboxylatbromid (Br-NDA) und hochmolekularer organischer Verunreinigungen, aus einer Lösung von roher 2,6-Naphthalindicarbonsäure (CNDA) mittels Hydrierung, wobei das Verfahren die Schritte umfasst:

(a) Berechnen einer Menge an Wasserstoff, welche erforderlich ist, um Verunreinigungen wie Formylnaphthalincarbonsäure, Naphthalincarboxylatbromid und hochmolekulare organische Verunreinigungen von schweren Substanzen zu entfernen;

(b) Einbringen einer festen Menge an Wasserstoff wie in Schritt (a) berechnet und Entfernen der Verunreinigungen mittels Hydrierung; und

(c) einmaliges Waschen des Produkts, das aus der Hydrierungsreaktion von Schritt (b) erhalten wurde, mit 200 bis 300°C heißem Wasser, um gereinigte 2,6-Naphthalindicarbonsäure herzustellen,

wobei die feste Menge an Wasserstoff gemäß einer folgenden Formel 1 berechnet wird

[Formel 1]

Menge an Wasserstoff, welche zur Entfernung von FNA erforderlich ist (ppm/h)

$$= \frac{[\text{CNDA-Eintrag (g/h)}] \times [\text{FNA-Gehalt (ppm)}] \times [2H_2(1/\text{mol})]}{[\text{MG von FNA (g/mol)}]}$$

Menge an Wasserstoff, welche zur Entfernung von Br-NDA erforderlich ist (ppm/h)

$$= \frac{[\text{CNDA-Eintrag (g/h)}] \times [\text{Br-NDA-Gehalt (ppm)}] \times [H_2(1/\text{mol})]}{[\text{MG von Br-NDA (g/mol)}]}$$

Menge an Wasserstoff, welche zur Entfernung schwerer Substanzen erforderlich ist (ppm/h)

$$= \frac{[\text{CNDA-Eintrag (g/h)}] \times [\text{Gehalt an schweren Substanzen (ppm)}] \times [1/2H_2(1/\text{mol})]}{[\text{MG von NDA (g/mol)}]}$$

2. Verfahren nach Anspruch 1, wobei die Hydrierungsreaktion bei 90 bis 130 kg/cm$^2$ und 290 bis 315°C in flüssiger Phase durchgeführt wird.

3. Verfahren nach Anspruch 1, wobei die Hydrierungsreaktion in Gegenwart eines Katalysators, der sich auf einem Träger von Aktivkohle befindet und 0,4 bis 0,6 Gewichts-% Palladium oder Platin, bezogen auf das Elementgewicht, als aktivierte Komponenten enthält, durchgeführt wird.

**Revendications**

1. Procédé d'élimination par hydrogénation d'impuretés incluant l'acide formylnaphtoïque, le bromure de naphtalènecarboxylate et les impuretés organiques de poids moléculaire élevé à partir d'une solution d'acide 2,6-naphtalènedicarboxylique (CNDA) brut, le procédé comprenant les étapes consistant à :

   (a) calculer une quantité d'hydrogène nécessaire pour éliminer les impuretés telles que l'acide formylnaphtoïque, le bromure de naphtalènecarboxylate et les impuretés organiques de poids moléculaire élevé de substances lourdes ;
   (b) introduire une quantité fixe d'hydrogène comme calculé dans l'étape (a) et éliminer les impuretés par hydrogénation ; et
   (c) laver le produit obtenu à partir de la réaction d'hydrogénation de l'étape (b) avec de l'eau chaude à 200 à 300 °C une fois pour produire de l'acide 2,6-naphtalènedicarboxylique purifié,

   dans lequel la quantité fixe d'hydrogène est calculée selon la formule 1 suivants :

Formule 1

Quantité d'hydrogène nécessaire pour l'élimination de FNA (ppm/h)

= [introduction de CNDA (g/h)] x [teneur en FNA (ppm)] x [2H$_2$ (1/mol)] / [MM de FNA (g/mol)]

Quantité d'hydrogène nécessaire pour l'élimination de Br-NDA (ppm/h)

= [introduction de CNDA (g/h)] x [teneur en Br-NDA (ppm)] x [H$_2$ (1/mol)] / [MM de Br-NDA (g/mol)]

Quantité d'hydrogène nécessaire pour l'élimination des substances lourdes (ppm/h)

= [introduction de CNDA (g/h)] x [teneur en substances lourdes (ppm)] x [1/2H$_2$ (1/mol)] / [MM de NDA (g/mol)]

2. Procédé selon la revendication 1, dans lequel la réaction d'hydrogénation est réalisée sous 90 à 130 kg/cm$^2$ et à 290 à 315°C dans une phase liquide.

3. Procédé selon la revendication 1, dans lequel la réaction d'hydrogénation est réalisée en présence d'un catalyseur supporté sur du charbon actif et contenant de 0,4 à 0,6 % en poids de palladium ou de platine sur la base du poids des éléments en tant que composants actifs.

Fig. 1

Fig. 2

Fig. 3

## REFERENCES CITED IN THE DESCRIPTION

**Patent documents cited in the description**

- US 5256817 A
- US 6756509 B
- US 6747171 B
- EP 1306361 A1
- WO 9929652 A